# EUROPEAN PATENT APPLICATION

(11) **EP 1 997 534 A1**
(43) Date of publication of application: **03.12.2008**
(21) Application number: 07290679.5
(22) Date of filing: 31.05.2007
(51) Int. Cl.: A61P 43/00, A61K 45/06, A61K 31/4745

(54) **Cancer treatment combination therapy comprising vinflunine and trastuzumab**

(71) Applicant: PIERRE FABRE MEDICAMENT, 92100 Boulogne-Billancourt (FR); F. Hoffmann-La Roche Ltd., 4070 Basel (CH)
(72) Inventor: The designation of the inventor has not yet been filed
(74) Representative: Ahner, Francis

(57) **Abstract**

The present invention relates to a therapeutic method for the treatment of cancer that comprises the use of a combination comprising vinflunine and trastuzumab.

## Description

The present invention deals with a method of treatment of cancer, more particularly breast cancer within people in need thereof, such a method comprising the steps of administration of vinflunine in combination with the steps of administration of trastuzumab.
Anticancer chemotherapies can entail the combined use of different agents, mainly in order to reduce the toxicity of one of the agents when used alone and in some cases because the combination may induce an increased efficacy as compared to each of the agents considered alone.

The study of anti-neoplastic properties of Vinca alkaloids allowed to show interesting activities of compounds comprising indol structure such as vincristine, vinblastine, or derivatives thereof such as vinflunine : 20',20'difluoro-3',4'-dihydrovinorelbine whose formula is described in EP 710 240.

The properties of vinflunine have been studied in vivo and the benefit of its use for the treatment of breast cancer has been established (Bennouna et al. 2003. Ann. Oncol. 14 : 630-637) and confirmed in a recent Phase II study (Campone et al. 2006. British Journal of Cancer. 95 : 1161-1166). Beside the observed efficacy of treatment with Vinflunine, the Overall Response of the selected patients as measured according to RECIST methodology was about 30% for breast cancer. The RECIST (Response Evaluation Criteria in Solid Tumors) methodology is a well documented and approved measurement directed to the evaluation of tumor response in case of chemotherapy (Duffaud, et al, "New guidelines to evaluate the response to treatment in solid tumors". Journal of the National Cancer Institute, 2000. Vol 92, N°3, 205-216.

Metastatic breast cancer (MBC) is essentially incurable with standard therapy, and patients with MBC have a median survival of about 2 years after documentation of metastasis. As a consequence, the goals of treatment are to improve patients' symptoms while trying to maintain (or improve, in certain cases) quality of life. Prolonging survival remains a clear goal, particularly in breast cancer that has overexpression or amplification of the her-2 oncogene.

Herceptin® (Trastuzumab) is an FDA-approved therapeutic monoclonal antibody for HER2 protein overexpressing metastatic breast cancer. Trastuzumab is currently approved by the FDA for the treatment of metastatic breast cancer that overexpresses HER2, (1) as a single agent after previous treatment of the metastatic breast cancer with one or more chemotherapy regimens and (2) in combination with paclitaxel in such patients without prior chemotherapy for their metastatic breast cancer. Moreover, there is evidence that the addition of trastuzumab to taxane adjuvant or neoadjuvant chemotherapy improves to patients with earlier stage breast cancer.

The present invention provides the use of combination therapies comprising vinflunine and trastuzumab in the treatment of neoplasm.
The invention further provides products containing trastuzumab and vinflunine formulated for simultaneous, separate or sequential use in treating neoplasms in a mammal.
The methods, regimens, combinations and products according to the present invention are useful in the treatment of a variety of neoplasms including lung cancers, including bronchioalveolar carcinoma and non small cell lung cancer, breast cancers, myeloma, prostate cancers, head and neck cancers, or transitional cell carcinoma; small cell and large cell neuroendocrine carcinoma of the uterine cervix.
In one embodiment, the combination of trastuzumab and vinflunine is particularly well suited for treatment of breast cancer, and particularly for metastatic breast cancer.

As used therein, the term vinflunine means 20',20'difluoro-3',4'-dihydrovinorelbine whose formula is described in EP 710 240, but it also encompasses its metabolites, such as deacetylvinflunine. Also included in the term vinflunine are pharmaceutically acceptable salts of vinflunine, such as vinflunine ditartrate for exemple.

As used in accordance with this invention, the term "treatment" means treating a mammal having a neoplasm by providing said mammal with an effective amount of a combination of vinflunine and trastuzumabwith the purpose of inhibiting progression of the neoplastic disease, growth of a tumor in such mammal, eradication of the neoplastic disease, prolonging survival of the mammal and/or palliation of the mammal.

The combinations of the invention may be in the form of a kit of parts. The invention therefore includes a product containing vinflunine and trastuzumab as a combined preparation for simultaneous, separate or sequential delivery for the treatment of a neoplasm in a mammal in need thereof. In one embodiment, a product contains vinflunine and trastuzumab as a combined preparation for simultaneous, separate or sequential use in treating a neoplasm in a mammal in need thereof. In one embodiment, the invention provides a pharmaceutical pack containing a course of an anti-neoplastic treatment for one individual mammal, wherein the pack contains (a) at least one unit of vinflunine and (b) at least one unit of trastuzumab in unit dosage form. In one embodiment, the neoplasm is metastatic breast cancer.

As is typical with oncology treatments, dosage regimens are closely monitored by the treating physician, based on numerous factors including the severity of the disease, response to the disease, any treatment related toxicities, age, and health of the patient. Dosage regimens are expected to vary according to the route of administration.
It is projected that initial i.v. infusion dosages of trastuzumab may be from about 2 to about 4 mg/kg/week, when administered on a weekly dosage regimen. In one embodiment the trastuzumab is delivered weekly and particularly for a treatment period of 3 weeks, i.e. administration of trastuzumab at Day 1 for the first week, Day 8 for the second week and day 15 for the third week. The treatment period may be repeated. The number of cycles may range from 3 to 20, preferably for about 3 to 19 times, more preferably for about 8 cycles.
According to the present invention, the expression "cycle" means a period of 3 weeks starting from Day 1 that comprises the first administered dosage. In one embodiment the dosage amount of the trastuzumab may be of about 2 mg/kg on day 1 or preferably 4 mg/kg on Day 1 as loading dosage, followed by 2mg/kg on day 8, for the second week and followed by 2 mg/kg on day 15 for the third week. This provides a therapeutic cycle of 3 weeks as of Day 1. This 3 weeks dosage regimen with one administration every week may be repeated.
For vinflunine, the projected i.v. dosage may vary between 250 mg/m2 to 320 mg/m2. In one embodiment, the dosage amount of vinflunine is about 320mg/m2. In an other embodiment the dosage amount of vinflunine is about 280 mg/m2. The dose regimen regarding vinflunine is therefore between 250 mg/m2 and 320 mg/m2, preferably about 320 mg/m2 on Day 1, as a loading dosage for 3 weeks. This administered dosage may be followed by the same dosage on day 22, thus making the starting of a new cycle ; Day 22 being the Day 1 of the second treatment cycle. Such 3 weeks cycle with one administration of vinflunine on Day 1 at the dosage of 250 mg/m2 to about 320 mg/mg, preferably 320 mg/m2 may be repeated from about 3 to 20 times, preferably for about 3 to 19 times, even preferably for about 8 times.
Administration of the compositions may be oral, intravenous, respiratory (e.g., nasal or intrabronchial), parenteral (besides i.v., such as intraperitoneal and subcutaneous injections), intraperitoneal, transdermal (including all administration across the surface of the body). Preferably, the administration of the compositions according to the combination of the present invention is done intravenously.
While the components of the invention may be delivered via the same route, a product or pack according to the invention may contain vinflunine, for delivery via a different route than that of the traztuzumab, e.g., one component may be delivered orally, while the other is administered intravenously. In another embodiment, vinflunine and traztuzumab are both delivered via the same route, e.g., i.v. Other variations would be apparent to one skilled in the art and are contemplated within the scope of the invention.
Preferred pharmaceutical forms suitable for injectable use include sterile aqueous solutions or dispersions and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms such as bacteria and fungi. In one embodiment, preferred injectable compositions for vinflunine are described in WO2005070425. Traztuzumab is commercially available from GENENTECH under the nam HERCEPTINE® as a sterile, white to pale yellow, preservative-free lyophilized powder for intravenous (IV) administration after reconstitution with bacteriostatic water for injection.

### EXEMPLE :

In an open-label phase I; thirty patients with medium age 55 (34-75) years were enrolled with their prior written consent. All the patients had histologically confirmed HER-2 positive breast carcinoma. Other eligibility criteria included :
- Evidence of progressive metastatic disease with at least one measurable lesion (according to RECIST evaluation methodology),
- No more than one prior chemotherapy for MBC,
- 18 years < age < 75 years,
- WHO performance status : 0 or 1
- Adequate bone marrow, hepatic and renal functions,
- Normal ECG and MUGA scan (LVEF≥ 50%).

The patients were given vinflunine 280 mg/m2 (9 patients) or 320 mg/m2 (21 patients) day 1 every 3 weeks(s) in combination with trastuzumab (loading dose 4 mg/kg on D1 and subsequently 2mg/kg/week starting on D8 and continued weekly for subsequent administrations).
At the first Vinflunine dose (280 mg/m2) 0/6 patients experienced dose-limiting toxicity. The recommended dose (RD) was established at 320 mg/m2 in the following 6 patients.

All 30 patients were evaluated for safety ; haematological and non-haematological toxicities were judged acceptable and no significant cardiac events were observed.

The efficacy was evaluated by an external radiologist according to RECIST methology. Such results are summarized in Table 1.

**TABLE 1 : Efficacy Results of the combination therapy.**

| | Vinflunine | | Vinflunine | |
|---|---|---|---|---|
| | 280 mg/m2 | | 320 mg/m2 | |
| | n | % | n | % |
| Complete Response (CR) | -- | -- | 1 | 4.8 |
| Partial Response (PR) | 6 | 66.7 | 15 | 71.4 |
| **Overall Response (OR) = CR+PR** | **6** | **66.7** | **16** | **76.2** |
| Stable Disease (SD) | 3 | 33.3 | 4 | 19 |
| Progressive Disease (PD) | -- | -- | 1 | 4.8 |
| Disease Control (CR+PR +SD) | 9 | 100 | 20 | 95.2 |

The overall Response (OR) is evaluated as 76.2 % in the case of Vinflunine (320 mg/m2, day 1 every 3 weeks) in combination with weekly trastuzumab with a loading dose 4mg/kg day 1 and subsequently 2 mg/kg/week), over a period of a median number of cycles of 8 cycles.

It is to be noted that the chemotherapy using vinflunine alone at the same dosage, at the same regimen and over the same period of time "produced" or "yielded" an Overall Response of about 30 % was observed (Campone et al. 2006. British Journal of Cancer. 95 : 1161-1166).
The observed OR, obtained by the combination therapy according to the present invention allows for an increase of 250 % of the OR as compared with vinflunine alone. An other very important parameter is the safety profile of this combination that was judged as very well tolerated with vinflunine Relative Dose Intensity of 89%.
Such results for the treatment of MBC with vinflunine/trastuzumab combination therapy is definitively a great advance in the domain of cancer therapy.

## Claims

1. A method of treating a neoplasm in a mammal in need thereof, which comprises providing to said mammal an effective amount of a combination of active components comprising trastuzumab and vinflunine.

2. A method of treating a neoplasm associated according to claim 1, wherein neoplasm is associated with overexpression or amplification of HER2.

3. The method according to any one of claims 1 or 2, wherein the neoplasm is selected from the group consisting of lung cancers, including bronchioalveolar carcinoma and non small cell lung cancer, breast cancers, myeloma, prostate cancers, head and neck cancer, or transitional cell carcinoma; small cell and large cell neuroendocrine carcinoma of the uterine cervix.

4. The method according to any one of claims 1 to 3, wherein the neoplasm is breast cancer.

5. The method according to claim 4, wherein the neoplasm is metastatic breast cancer.

6. A regimen for treatment of cancer, said regimen comprising:
delivering a dosage amount of vinflunine; and
delivering a dosage amount of trastuzumab .

7. A regimen according to claim 6, wherein the cancer is associated with overexpression or amplification of HER2.

8. A regimen according to claim 6 or 7, wherein the neoplasm is selected from the group consisting of lung cancers, including bronchioalveolar carcinoma and non small cell lung cancer, breast cancers, myeloma, prostate cancers, head and neck cancer, or transitional cell carcinoma; small cell and large cell neuroendocrine carcinoma of the uterine cervix.

9. A regimen according to any one of claims 6 to 8, wherein the cancer is breast cancer.

10. A regimen according to any one of claims 6 to 9, wherein the cancer is metastatic breast cancer.

11. The regimen according to claim 6, wherein the vinflunine and/or the trastuzumab is delivered intravenously.

12. The regimen according to claim 6, wherein the trastuzumab is delivered weekly.

13. The regimen according to claim 6, wherein the vinflunine is delivered once every three weeks.

14. The regimen according to claim 12, wherein the trastuzumab is delivered weekly over a period of three weeks.

15. The regimen according to claim 12, wherein the trastuzumab is delivered weekly over a period of 6 to 54 weeks, preferably 24 weeks.

16. The regimen according to claim 13, wherein the vinflunine is delivered once every three weeks over a period of 6 to 54 weeks, preferably 24 weeks.

17. The regimen according to claim 13, wherein the vinflunine is delivered over a period of 24 weeks.

18. The regimen according to any one of claims 6 to 17, wherein the dosage amount of vinflunine is 320 mg/m2 once every three weeks.

19. The regimen according to any one of claims 6 to 18, wherein the dosage amount of trastuzumab is 2 mg/kg/week.

20. A product containing vinflunine and trastuzumab as a combined preparation for simultaneous, separate or sequential use in treating a neoplasm in a mammal.

21. A pharmaceutical pack containing a course of an anti-neoplastic treatment for one individual mammal, wherein the pack contains (a) at least one unit of vinflunine and (b) at least one unit of trastuzumab in unit dosage form.
